# EUROPÄISCHE PATENTSCHRIFT

(11) **EP 3 559 464 B1**
(45) Veröffentlichungstag und Bekanntmachung des Hinweises auf die Patenterteilung: **25.11.2020**
(21) Anmeldenummer: 17826217.6
(22) Anmeldetag: 19.12.2017
(51) Int. Cl.: F04B 43/02, F04B 43/073

(54) **MEMBRANPUMPENEINRICHTUNG UND MEMBRANPUMPE MIT EINER MEMBRANPUMPENEINRICHTUNG UND EINER BETÄTIGUNGSEINRICHTUNG**
DIAPHRAGM PUMP DEVICE AND DIAPHRAGM PUMP HAVING A DIAPHRAGM PUMP DEVICE AND AN ACTUATION DEVICE
DISPOSITIF DE POMPE À MEMBRANE ET POMPE À MEMBRANE COMPRENANT UN DISPOSITIF DE POMPE À MEMBRANE ET UN DISPOSITIF D'ACTIONNEMENT

(30) Priorität: 21.12.2016 DE 102016015206
(43) Veröffentlichungstag der Anmeldung: 30.10.2019
(73) Patentinhaber: Fresenius Medical Care Deutschland GmbH, 61352 Bad Homburg v.d.H (DE)
(72) Erfinder: ANDERLE, Jens, 73326 Deggingen (DE)
(74) Vertreter: Oppermann, Frank
(86) Internationale Anmeldenummer: PCT/EP2017/083665
(87) Internationale Veröffentlichungsnummer: WO 2018/115028

(56) Entgegenhaltungen:
- WO-A1-2004/090334
- DE-A1- 19 720 482
- DE-B- 1 013 522
- US-A- 2 383 193
- US-A- 4 050 859
- US-A- 5 195 986
- US-A1- 2013 178 752

## Beschreibung

Die Erfindung betrifft eine Membranpumpeneinrichtung zur Förderung von Fluiden, insbesondere von medizinischen Flüssigkeiten für eine Blutbehandlung. Darüber hinaus betrifft die Erfindung eine Membranpumpe mit einer Membranpumpeneinrichtung und einer Betätigungseinrichtung für die Membranpumpeneinrichtung.

Zur Förderung von medizinischen Flüssigkeiten finden in der Medizintechnik Membranpumpen Verwendung. Der Vorteil von Membranpumpen liegt in der Trennung der Antriebseinheit von dem zu fördernden Fluid durch eine Membran.

In der Medizintechnik werden an Membranpumpen hohe Anforderungen gestellt. Für bestimmte Anwendungen sollen die Membranpumpen auch bei niedrigen Flüssen eine hohe Fördergenauigkeit haben. Eine besonders genaue Dosierung bei einer niedrigen Flussrate erfordert beispielsweise die Citratantikoagulation (CiCa-Antikoagulation) in der Dialyse.

Es sind Membranpumpen bekannt, die über eine nur zur einmaligen Verwendung bestimmte Membranpumpeneinrichtung verfügen. Die Membranpumpeneinrichtung dieser Membranpumpen kann als eine Einwegkassette ausgebildet sein, in der eine Ausnehmung ausgebildet ist, die von einer elastischen Membran verschlossen ist. Während der Saugphase wird die Fluidströmung aus der Pumpenkammer unterbrochen und während der Druckphase wird die Fluidströmung in die Pumpenkammer unterbrochen. Der Antrieb der elastischen Membran kann mit einer Betätigungseinrichtung erfolgen, die nicht zur einmaligen Verwendung bestimmt ist.

Die US 2013/0178752 A1 betrifft eine Vorrichtung zur Steuerung einer gasförmigen Fluidströmung, welche für eine Druckpumpe zum Befüllen einer Manschette eines Blutdruckmessgerätes mit Druckluft bestimmt ist, wobei die Druckpumpe insbesondere eine piezoelektrische Vibrationspumpe ist. Die Druckpumpe und die Steuervorrichtung bilden eine bauliche Einheit, die einen Einlass und einen Anschluss einer zu der Manschette führenden Druckleitung sowie einen Auslass aufweist. Darüber hinaus umfasst die Steuervorrichtung ein erstes Ventil und ein zweites Ventil. Beide Ventile verfügen jeweils über einen Ventilkörper, in dem eine Kammer ausgebildet ist, die von einer elastischen Membran in einen ersten Ventilraums und einen zweiten Ventilraum unterteilt ist. In der Kammer ist ein Ventilsitz angeordnet, dessen Stirnseite der Membran zugewandt ist, wobei der Ventilsitz von einem Ventilkanal durchsetzt ist. Die Querschnittsfläche des Ventilsitzes ist kleiner als die Querschnittsfläche des den Ventilsitz umschließenden Bereichs der Kammer. Ein erster Fluidkanal verbindet den Auslass der Druckpumpe mit dem Einlass des ersten Ventils und ein zweiter Fluidkanal verbindet den Auslass des ersten Ventils mit dem Anschluss für die Druckleitung.

Die DE 197 20 482 A1 beschreibt eine Membranpumpe, die eine von einem piezoelektrischen Aktor betätigte Pumpenmembran und ein als Membranventil ausgebildetes Einlassventil, das im Zustrompfad vorgesehen ist, und ein als Membranventil ausgebildetes Auslassventil, das im Abstrompfad vorgesehen ist, aufweist. Einlass- und Auslassventil weisen einen Ventilsitz in einer Ausnehmung eines Ventilkörpers auf.

Die US 5,195,986 A beschreibt ein automatisches Abschaltventil für eine Fluidleitung. Das Abschaltventil weist einen Ventilsitz mit einem Durchgang auf, der von einer Membran verschlossen wird.

Die WO 2004/090334 offenbart eine medizinische Membranpumpe mit Membranventilen, welche jeweils einen Raum aufweisen, der mittels Kanälen einerseits mit dem Pumpenraum verbunden ist und andererseits mit dem Pumpenausgang respektive Pumpeneingang.

Der Erfindung liegt die Aufgabe zugrunde, eine Membranpumpe der oben beschriebenen Bauart derart weiterzubilden, dass die Membranpumpe eine besonders genaue Dosierung von Fluiden, beispielsweise einer Lösung zur CiCa-Antikoagulation, auch bei sehr kleinen Flussraten erlaubt, wobei ein konstanter Fluidmassenstrom gewährleistet werden soll. Insbesondere liegt der Erfindung die Aufgabe zugrunde, die Fördergenauigkeit der

Membranpumpeneinrichtung einer derartigen Membranpumpe zu verbessern.

Eine weitere Aufgabe der Erfindung ist, eine Blutbehandlungsvorrichtung, insbesondere eine Dialysevorrichtung, mit einer Membranpumpe bereitzustellen, die eine besonders genaue Dosierung eines Fluids, beispielsweise einer Lösung zur CiCa-Antikoagulation, auch bei sehr kleinen Flussraten mit einem konstanter Fluidmassenstrom erlaubt.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Membranpumpeneinrichtung weist einen Pumpenkammerkörper auf, in dem eine Ausnehmung ausgebildet ist, die zur Bildung einer Pumpenkammer von einer elastischen Membran verschlossen ist. Darüber hinaus umfasst die Membranpumpeneinrichtung einen Zustrompfad, der einen Eingangsanschluss mit einer Einlassöffnung der Pumpenkammer verbindet, und einen Abstrompfad, der eine Auslassöffnung der Pumpenkammer mit einem Ausgangsanschluss verbindet. In dem Zustrompfad ist ein Einlassventil und in dem Abstrompfad ist ein Auslassventil vorgesehen, um die Flüssigkeitsströmung in dem Zustrom- bzw. Abstrompfad beeinflussen zu können.

Die Erfindung betrifft insbesondere die Ausgestaltung des Auslassventils auf der Druckseite der Membranpumpeneinrichtung. Bei der erfindungsgemäßen Membranpumpeneinrichtung ist das Auslassventil ein Membranventil, das einen Ventilkörper aufweist, in dem eine Ausnehmung ausgebildet ist, die zur Bildung eines Ventilraums von einer elastischen Membran verschlossen ist, in welchem ein Ventilsitz angeordnet ist, dessen Stirnseite der Membran zugewandt ist. In der Öffnungsstellung des Auslassventils ist die Membran im Abstand zu dem Ventilsitz angeordnet, wobei der Ventilsitz von einem Ventilkanal durchsetzt ist. In der Schließstellung sitzt die Membran auf dem Ventilsitz auf, so dass das Auslassventil geschlossen ist.

Die elastische Membran der Pumpenkammer und die elastische Membran des Auslassventils können eine gemeinsame Membran der Membranpumpeneinrichtung sein. Es können aber auch getrennte Membranen vorgesehen sein.

Zur Erzielung eines konstanten kleinen Flusses ist eine Regelung der Flüssigkeitsströmung erforderlich. Eine derartige Regelung hat sich bei der Entwicklung einer Membranpumpeneinrichtung, deren Auslassventil ein Membranventil ist, aber als besonders schwierig erwiesen. In Versuchen hat sich gezeigt, dass das System zum Schwingen neigt.

Die Erfindung setzt voraus, dass in der Ruhestellung auf die Membran des Auslassventils von einer Betätigungseinrichtung ein Anpressdruck aufgebracht wird, so dass die Membran den Ventilkanal verschließt. Beispielsweise kann der Druck von einem federnd vorgespannten Stößel aufgebracht werden.

Bei der erfindungsgemäßen Membranpumpeneinrichtung wird das Regelverhalten dadurch entscheidend verbessert, dass der Abstrompfad einen ersten Abstromkanal umfasst, der die Auslassöffnung der Pumpenkammer mit einer Einlassöffnung des Ventilraums des Auslassventils verbindet, und einen zweiten Abstromkanal umfasst, der den Auslassventilkanal mit dem Ausgangsanschluss verbindet, wobei die Querschnittsfläche des Auslassventilsitzes kleiner als die Querschnittsfläche des den Auslassventilsitz umschließenden Bereichs des Auslassventilraums ist. Diese Anordnung bewirkt eine "mechanische Gegenkopplung", die für die Regelung der Fluidströmung von Vorteil ist.

Zu Beginn des Pumpenschlags steht das Fluid in der Pumpenkammer unter Druck. Da die Pumpenkammer über den ersten Abstromkanal mit der Einlassöffnung des Ventilraums des Auslassventils in Fluidverbindung steht, wird die äußere Fläche der Membran, die den Ventilsitz umschließt, von dem Fluid mit Druck beaufschlagt. Die innere Fläche der Membran wird hingegen nicht mit Druck beaufschlagt, da die Membran mit der inneren Fläche auf dem Ventilsitz sitzt. Mit dem Pumpenschlag muss die Federkraft des federnd vorgespannten Stößels, der die Membran auf den Ventilsitz drückt, überwunden werden. Sobald die Membran von dem Ventilsitz abgehoben ist, wird auch die innere Fläche der Membran von dem Fluid mit Druck beaufschlagt. Da die innere Fläche der Membran aber kleiner als die äußere Fläche ist, was sich daraus ergibt, dass die Querschnittsfläche des Auslassventilsitzes kleiner als die Querschnittsfläche des den Ventilsitz umschließenden Bereichs des Ventilraums ist, nimmt die Kraft, mit der die Membran von dem Ventilsitz gedrückt wird, aufgrund der relativ geringen Änderung der wirksamen Fläche nur geringfügig zu, so dass bei abnehmenden Pumpendruck die Rückstellkraft des federnd vorgespannten Stößels schnell wieder überwiegt. Es hat sich in Versuchen gezeigt, dass das System dann nicht zum Schwingen neigt.

Für das Öffnungsverhalten des Auslassventils ist die Form des Ventilsitzes nicht ausschlaggebend. Eine bevorzugte Ausführungsform der Erfindung sieht vor, dass der Ventilsitz des Auslassventils ein ringförmiger Ventilsitz ist.

Die Ausbildung des Einlassventils ist für das Öffnungsverhalten des Auslassventils nicht entscheidend. Eine bevorzugte Ausführungsform der Erfindung sieht vor, dass auch das Einlassventil ein Membranventil ist, wobei beide Ventile vorzugsweise baugleich sind. Das Einlassventil weist vorzugsweise einen Einlassventilkörper auf, in dem eine Ausnehmung ausgebildet ist, die zur Bildung eines Einlassventilraums von einer elastischen Membran verschlossen ist, in welchem ein Einlassventilsitz angeordnet ist, dessen Stirnseite der Membran zugewandt und in der Öffnungsstellung des Einlassventils im Abstand zu der Membran angeordnet ist, wobei der Einlassventilsitz von einem Einlassventilkanal durchsetzt ist. Pumpenkammer und Einlass- und Auslassventil können wiederum eine gemeinsame elastische Membran haben.

Bei einer bevorzugten Ausführungsform ist die Membranpumpeneinrichtung oder ein Teil der Membranpumpeneinrichtung als eine zur einmaligen Verwendung bestimmte Kassette ausgebildet, die für die Verwendung mit einer Betätigungseinrichtung der Membranpumpe bestimmt ist. Für das Öffnungsverhalten des Auslassventils ist aber die Ausbildung der Membranpumpeneinrichtung oder eines Teil derselben als Einwegkassette nicht wesentlich. Membranpumpeneinrichtung und Betätigungseinrichtung können auch im Gebrauch nicht voneinander trennbare Einrichtungen sein.

Bei einer besonders bevorzugten Ausführungsform sind der Pumpenkammerkörper und der Einlassventil- und Auslassventilkörper Bestandteil eines einteiligen oder mehrteiligen Gehäusekörpers der Kassette, wobei die Membran der Pumpenkammer und die Membran des Einlass- und Auslassventils an einer Anlagefläche angeordnet sind, die an eine Montagefläche der Betätigungseinrichtung ankoppelbar ist.

Die erfindungsgemäße Membranpumpe umfasst die erfindungsgemäße Pumpeneinrichtung und eine Betätigungseinrichtung, die derart ausgebildet ist, dass die Membran der Pumpenkammer zwischen einer Ansaugkonfiguration und einer Austragkonfiguration deformierbar wird, wobei das Einlass- und Auslassventil wechselweise öffenbar und schließbar sind, so dass von der Membranpumpe Fluid förderbar ist.

Die Vorteile der Erfindung kommen insbesondere bei einer Membranpumpe zum Tragen, deren Betätigungseinrichtung vorzugsweise eine Auslass-Stelleinrichtung aufweist, die ein Auslassstellglied zur Deformation der Membran des Auslassventils aufweist, das in eine Ruheposition federnd vorgespannt ist, in der die Membran des Auslassventils auf dem Auslassventilsitz sitzt, so dass das Auslassventil geschlossen ist, wobei das Auslassstellglied aus der Ruheposition in eine Funktionsstellung bewegbar ist, in der die Membran des Auslassventils im Abstand zu dem Auslassventilsitz angeordnet ist, so dass das Auslassventil geöffnet ist.

Für die Betätigung des Einlassventils weist die Betätigungseinrichtung vorzugsweise eine entsprechende Einlass-Stelleinrichtung auf, die ein Einlassstellglied zur Deformation der Membran des Einlassventils aufweist, das in eine Ruheposition federnd vorgespannt ist, in der die Membran des Einlassventils auf dem Einlassventilsitz sitzt, so dass das Einlassventil geschlossen ist, wobei das Einlassstellglied aus der Ruheposition in eine Funktionsstellung bewegbar ist, in der die Membran des Einlassventils im Abstand zu dem Einlassventilsitz angeordnet ist, so dass das Einlassventil geöffnet ist.

Weiterhin verfügt die Betätigungseinrichtung vorzugsweise über eine erste Arbeitsfluidleitung zur Versorgung der Einlass-Stelleinrichtung mit einem Arbeitsfluid zur Betätigung des Einlassstellgliedes und eine zweite Arbeitsfluidleitung zur Versorgung der Auslass-Stelleinrichtung mit einem Arbeitsfluid zur Betätigung des Auslassstellgliedes, und die Einlass-Stelleinrichtung verfügt vorzugsweise über ein erstes Arbeitsfluidventil zur Beeinflussung eines Querschnitts der ersten Arbeitsfluidleitung und die Auslass-Stelleinrichtung über ein zweites Arbeitsfluidventil zur Beeinflussung eines Querschnitts der zweiten Arbeitsfluidleitung, wobei das zweite Arbeitsfluidventil als Proportionalventil und das erste Arbeitsfluidventil als Schaltventil ausgeführt sind.

Eine besonders bevorzugte Ausführungsform sieht vor, dass die Betätigungseinrichtung eine Steuereinrichtung aufweist, mit der das erste und zweite Arbeitsfluidventil elektrisch verbunden sind, wobei die Steuereinrichtung derart konfiguriert ist, dass in Abhängigkeit von dem Fluidmassenstrom der Membranpumpeneinrichtung ein Drucksollwert für das zweite Arbeitsfluidventil derart bestimmt wird, dass der Fluidmassenstrom während der Austragsphase der Membranpumpeneinrichtung konstant ist.

Die Betätigungseinrichtung weist vorzugsweise eine Steuerkammer und eine Steuerfluidleitung zur Beaufschlagung der Steuerkammer mit einem Steuerfluid auf, in welcher ein Steuerfluidventil angeordnet ist, das zur Veränderung eines Querschnitts der Steuerfluidleitung ausgebildet ist, wobei die Membran der Pumpenkammer durch Beaufschlagung mit dem Steuerfluid in eine Ansaugkonfiguration oder eine Austragkonfiguration deformierbar ist. Zur Ermittlung des Fluidmassenstroms ist vorzugsweise ein Steuerfluid-Drucksensor vorgesehen, der elektrisch mit der Steuereinrichtung verbunden ist, wobei die Steuereinrichtung derart konfiguriert ist, dass der Fluidmassenstrom in Abhängigkeit von dem Drucksignal des Steuerfluid-Drucksensors ermittelt wird.

Die Membranpumpe kann vorteilhafterweise in der Medizintechnik verwendet werden. Vorteile ergeben sich aber auch bei einer Verwendung in anderen Bereichen der Technik. Eine besonders bevorzugte Verwendung ist der Einsatz der Membranpumpe in einer Blutbehandlungsvorrichtung, insbesondere Dialysevorrichtung, mit einem Behältnis zur Bereitstellung einer medizinischen Flüssigkeit, insbesondere einer Antikoagulations-Lösung.

Nachfolgend wird ein Ausführungsbeispiel einer Membranpumpe, die eine Pumpeneinrichtung und eine Betätigungseinrichtung umfasst, im Einzelnen beschrieben.

Es zeigen:
- Fig. 1: eine Schnittdarstellung eines Teils einer Betätigungseinrichtung mit einer Steuerkammer und einer Stelleinrichtung,
- Fig. 2: eine Schnittdarstellung einer Membranpumpeneinrichtung,
- Fig. 3: ein schematisches Schaltbild der Betätigungseinrichtung gemäß der Fig. 1,
- Fig. 4: ein Steuerdiagramm für die Betätigungseinrichtung gemäß der Fig. 1 und
- Fig. 5: eine schematische Darstellung des erfindungsgemäßen Auslassventils,
- Fig. 6: eine schematische Darstellung einer anderen Auslassventilanordnung,
- Fig. 7A: die auf das Auslassventil wirkende Kraft in Öffnungsrichtung bei dem erfindungsgemäßen Auslassventil mit einer Regelung,
- Fig. 7B: die auf das Auslassventil wirkende Kraft in Öffnungsrichtung bei dem erfindungsgemäßen Auslassventil ohne Regelung,
- Fig. 7C: die auf das Auslassventil wirkende Kraft in Öffnungsrichtung bei einer anderen Auslassventilanordnung,
- Fig. 8A: der zeitliche Verlauf der Kräfte für den Fall einer "mechanischen Gegenkopplung" und
- Fig. 8B: der zeitliche Verlauf der Kräfte für den Fall einer "mechanischen Mitkopplung".

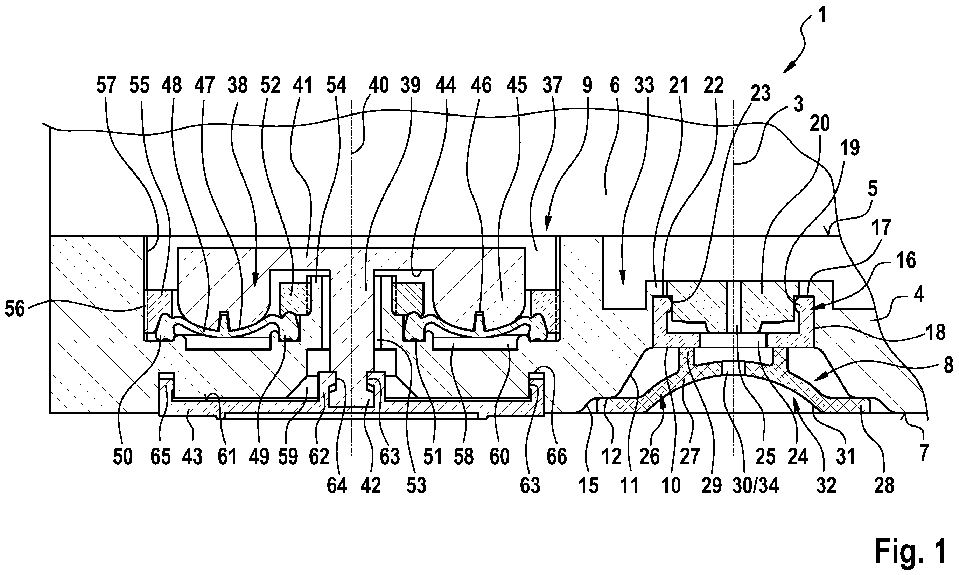

Zunächst wird unter Bezugnahme auf die Figuren 1 bis 4 ein Ausführungsbeispiel der erfindungsgemäßen Membranpumpe beschrieben, die über eine Betätigungseinrichtung und eine Membranpumpeneinrichtung verfügt, wobei sämtliche Baugruppen der Membranpumpe im Einzelnen beschrieben werden.

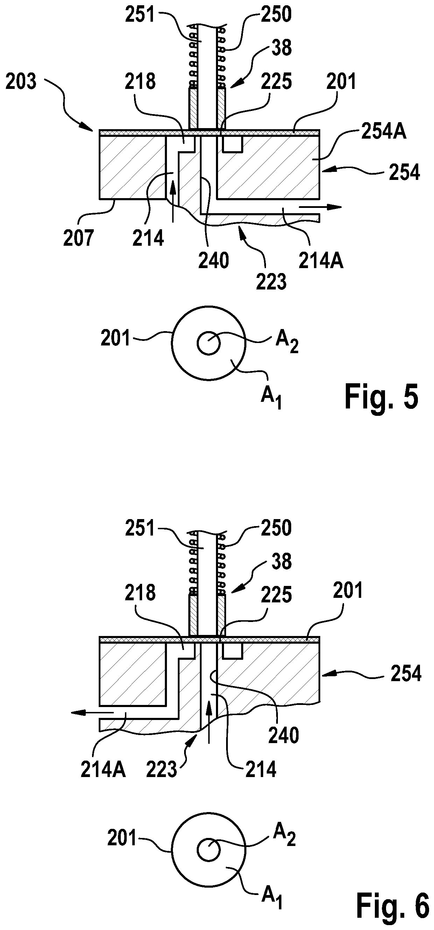

Die für die Erfindung besonders wesentlichen Komponenten der Membranpumpe werden unter Bezugnahme auf die Figuren 5 und 6 sowie die Figuren 7A bis 7C anhand von schematischen Darstellungen des Auslassventils und anhand des zeitlichen Verlaufs der auf die Membran des Ventils in Öffnungsrichtung wirkenden Kraft im Vergleich zu einer anderen Auslassventilanordnung beschrieben.

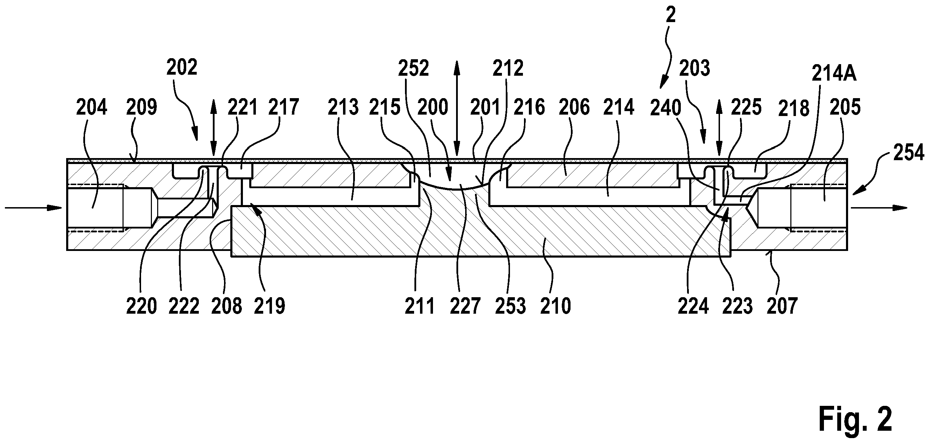

Die in der Fig. 1 dargestellte Betätigungseinrichtung 1 ist für eine mechanische Ansteuerung einer in Fig. 2 dargestellten Membranpumpeneinrichtung 2 vorgesehen, wobei ein nicht dargestellter mechanischer Verbund aus der Betätigungseinrichtung 1 und der Membranpumpeneinrichtung 2 eine Fluidförderung eines vorzugsweise flüssigen Fluids bei vollständiger Trennung des zu fördernden Fluids von der Betätigungseinrichtung 1 ermöglicht.

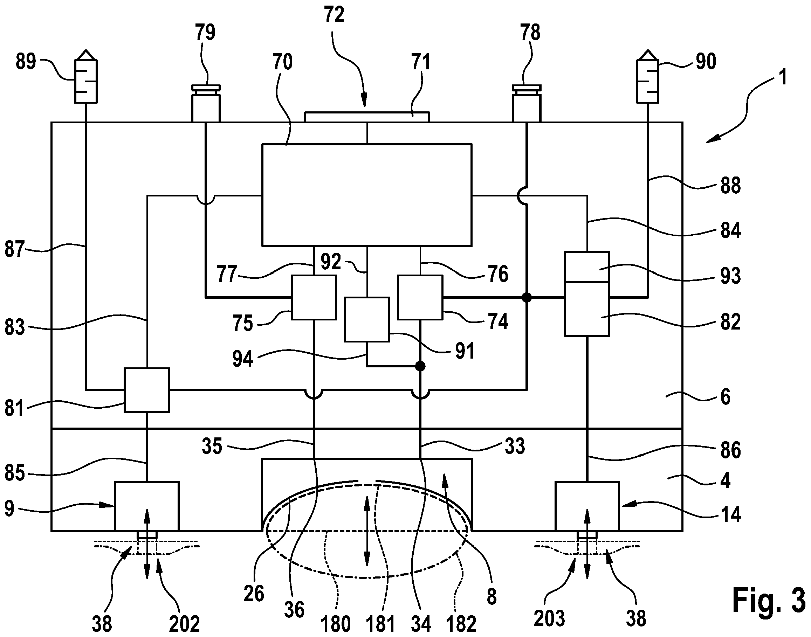

Die Betätigungseinrichtung 1 ist gemäß der Darstellung der Fig. 1 rein exemplarisch spiegelsymmetrisch zu einer Spiegelebene ausgebildet, die senkrecht zur Darstellungsebene der Fig. 1 ausgerichtet ist und die eine Mittelachse 3 umfasst. Die Betätigungseinrichtung 1 umfasst einen Grundkörper 4, der beispielhaft quaderförmig ausgebildet ist und der insbesondere als Kunststoffspritzgussteil hergestellt sein kann. An einer beispielhaft eben ausgebildeten Oberseite 5 des Grundkörpers 4 ist eine Steuerbaugruppe 6 angebracht, die nachstehend im Zusammenhang mit der Fig. 3 näher beschrieben wird. Eine exemplarisch zumindest im Wesentlichen eben ausgebildete Unterseite des Grundkörpers 4 dient als Montagefläche 7 für die in Fig. 2 dargestellte und ebenfalls nachstehend näher beschriebene Membranpumpeneinrichtung 2.

Wie der teilweise geschnittenen Darstellung der Fig. 1 entnommen werden kann, sind im Grundkörper 4 eine Steuerkammer 8 sowie eine Stelleinrichtung 9 angeordnet. Dabei ist die Steuerkammer 8 für eine Betätigung einer Membran 201 einer Membranpumpe 200, die in der Membranpumpeneinrichtung 2 ausgebildet ist, vorgesehen. Die Stelleinrichtung 9 dient zur Ansteuerung eines Membranventils 202, 203, das ebenfalls in der Membranpumpeneinrichtung 2 ausgebildet ist.

Die Steuerkammer 8 ist beispielhaft als rotationssymmetrische Ausnehmung in der Montagefläche 7 ausgebildet, wobei die Mittelachse 3 als Rotationsachse für ein Profil der Steuerkammer 8 vorgesehen ist. Die Steuerkammer 8 ist im Wesentlichen mit einem napfförmigen Querschnitt versehen und weist einen kreisrunden Bodenbereich 10, einen konusabschnittsförmigen Wandbereich 11, eine kreisringförmige Kontaktfläche 12 und einen daran angrenzenden, konusabschnittsförmigen Auslaufbereich 15 auf. Der Bodenbereich 10 wird beispielhaft von einer Oberfläche eines kreisringförmigen Dichtelements 16 gebildet, das in einer an die Steuerkammer 8 angrenzenden Ausnehmung 17 eingesetzt ist und das bezogen auf die Mittelachse 3 ein L-förmiges Profil aufweist. Das Dichtelement 16 liegt mit einer kreiszylindrischen Außenoberfläche 18 flächig an einer gegenüberliegenden, nicht bezeichneten inneren Oberfläche der Ausnehmung 17 an und weist an einem kürzeren L-Schenkel einen radial nach innen weisenden Ringbund 19 auf, der für eine abdichtende Anlage an einer Außenoberfläche eines rotationssymmetrisch zur Mittelachse 3 ausgebildeten Kanalteils 20 vorgesehen ist. Um eine eindeutige Platzierung des Dichtelements 16 und des Kanalteils 20 zu gewährleisten weist die Ausnehmung 17 einen radial nach innen vorspringenden Ringbund 21 auf, an dem eine axiale, kreisringförmige Stirnseite 22 des Dichtelement 16 formschlüssig anliegt. Das Kanalteil 20 liegt seinerseits mit einer Stufe 23 an der Stirnseite 22 an. Sowohl das Dichtelement 16 als auch das Kanalteil 20 sind jeweils von einer zentrischen Ausnehmung 24, 25 durchsetzt, die als Bestandteil einer Steuerfluidleitung 33 anzusehen sind.

In der Steuerkammer 8 ist ein Formkörper 26 aufgenommen, der rein exemplarisch rotationssymmetrisch zur Mittelachse 3 ausgebildet ist und umfasst einen im Wesentlichen als Kugelschalenabschnitt ausgeführten Hauptkörper 27, einen hiervon radial nach außen abragenden Ringbund 28 und einen koaxial zur Mittelachse 3 ausgerichteten Stützring 29. Der Hauptkörper 27 ist von einer zentral angeordneten Ausnehmung 30 durchsetzt und bildet an einer dem Stützring 29 abgewandten Oberfläche eine als Kugelkalotte geformte Anlagefläche 31 aus.

Der Ringbund 28 liegt an der Kontaktfläche 12 der Steuerkammer 8 an, während der Stützring 29 stirnseitig am Bodenbereich 10 der Steuerkammer 8 abgestützt ist. Die Anlagefläche 31 dient als Begrenzung für einen Raumabschnitt, der als Membranarbeitsbereich 32 bezeichnet wird und begrenzt bei Anbringung der Membranpumpeneinrichtung 2 an der Betätigungseinrichtung 1 eine Deformation der Membran 201 der Membranpumpe 200. Die Ausnehmung 30 im Formkörper 26 dient als Steuerfluidmündung 34.

Benachbart zur Steuerkammer 8 ist im Grundkörper 4 die Stelleinrichtung 9 angeordnet, bei der es sich beispielhaft um einen fluidisch betreibbaren Linearaktor handelt. Exemplarisch umfasst die Stelleinrichtung 9 ein linearbeweglich in einer Ausnehmung 37 aufgenommenes Stellglied 38, das zur Betätigung des Membranventils 202, 203 der Membranpumpeneinrichtung 2 vorgesehen ist. Das Stellglied 38 wird beispielhaft ohne eine Bereitstellung von druckbeaufschlagtem Fluid durch Einwirkung einer nicht dargestellten Feder in eine nicht dargestellte Ruheposition verlagert. Gemäß der Darstellung der Fig. 1 befindet sich das Stellglied 38 durch Bereitstellung von druckbeaufschlagtem Fluid in einer Funktionsposition. Das Stellglied 38 umfasst einen zylindrischen Stößel 39, dessen Längsachse 40 quer zur Montagefläche 7 ausgerichtet ist. Der Stößel 39 ist mit einem hinteren Endbereich an einer Verbindungsplatte 41 angebracht und ist mit einem vorderen Endbereich 42 an einer tellerförmig ausgebildeten Dichtmembran 43 festgelegt. Die Verbindungsplatte 41 ist rein exemplarisch kreisscheibenförmig ausgebildet und weist an einer Vorderseite 44, an der der Stößel 39 mittig angebracht ist, in einem radial außen liegenden Bereich einen kreisringförmigen Stützring 45 auf, der in die gleiche Richtung wie der Stößel 39 abragt. Wie aus der Darstellung der Fig. 1 entnommen werden kann, weist der Stützring 45 einen U-förmigen Querschnitt mit einer zentral angeordneten, umlaufenden Nut 46 auf und liegt mit einer der Vorderseite 44 abgewandten, konvex gekrümmten Stützfläche 47 an einem Dichtring 48 an.

Der Dichtring 48 ist koaxial zum Stößel 39 und rotationssymmetrisch zur Längsachse 40 ausgebildet und weist radial innenliegend sowie radial außenliegend jeweils einen ringförmigen Dichtwulst 49, 50 auf. Der innenliegende Dichtwulst 49 ist in einer nutförmigen, umlaufenden Vertiefung 51 im Grundkörper 4 aufgenommen und wird rein exemplarisch von einem Schraubring 52, der auf einen koaxial zur Längsachse 40 ausgebildeten und mit einer Ausnehmung 53 für den Stößel 39 versehenen Schraubstutzen 54 des Grundkörpers 4 aufgeschraubt ist, am Grundkörper 4 festgelegt. In gleicher Weise wird der außen liegende Dichtwulst 50 von einem Schraubring 55, der mit einem Außengewinde 56 in ein am Grundkörper 4 ausgebildetes Innengewinde 57 eingeschraubt ist, ebenfalls am Grundkörper 4 festgelegt.

In einem Bereich zwischen dem inneren Dichtwulst 49 und dem äußeren Dichtwulst 50 ist gegenüberliegend zum Stützring 45 im Grundkörper 4 eine ringförmige Ausnehmung 60 eingebracht, die eine Linearbewegung des Stellglieds 38 entlang der Längsachse 40 und gemäß der Darstellung der Fig. 1 nach unten ermöglicht. Der abdichtend am Grundkörper 4 festgelegte Dichtring 48 bestimmt zusammen mit der ringförmigen Ausnehmung 60 einen Arbeitsraum, der einen Endbereich eines Arbeitsfluidkanals 58 darstellt und mit druckbeaufschlagtem Fluid versorgt werden kann. Bei einer solchen Versorgung dieses Arbeitsraums wird das Stellglied aufgrund einer elastischen Deformation des Dichtrings 48 aus einer nicht dargestellten Ruheposition in die Funktionsposition gemäß der Fig. 1 verlagert. Hier-durch wird auch die Dichtmembran 43 deformiert, die aus einer von der Montagefläche 7 abragenden Stellung in die zumindest im Wesentlichen oberflächenbündige Stellung gemäß der Fig. 1 gebracht werden kann. Dabei ist vorgesehen, dass die Dichtmembran 43 in der Ruhestellung an dem jeweils zugeordneten Membranventil 202, 203 der Membranpumpeneinrichtung 2 anliegt, um das Membranventil 202, 203 aus einer Öffnungsstellung in eine Schließstellung zu bringen. Bei der Beaufschlagung des Arbeitsraums mit druckbeaufschlagtem Fluid findet durch die damit verbundene Verlagerung des Stellglieds 38 in die Funktionsposition gemäß der Fig. 1 eine Öffnungsbewegung 30 für das jeweils zugeordnete Membranventil 202, 203 der Membranpumpeneinrichtung 2 statt, wie dies schematisch auch in der Fig. 3 dargestellt ist. Die Dichtmembran 43 weist an einer dem Stößel 39 zugewandten Oberseite 61 einen Ringbund 62 auf, der mit einem radial nach innen weisenden Vorsprung 63 versehen ist, der formschlüssig in eine am vorderen Endbereich 42 ausgebildete Ringnut 64 eingreift. Durch diese formschlüssige Verbindung zwischen Stößel 39 und Dichtmembran 43 wird eine bidirektionale Krafteinleitung vom Stößel 39 auf die Dichtmembran 43 und umgekehrt ermöglicht. In einem radial außenliegenden Umfangsbereich der Dichtmembran 43 ist diese mit einem koaxial abragenden, einstückig angeformten Haltering 65 versehen, der in einer im Grundkörper 4 koaxial zur Längsachse 40 nutartig eingebrachte Ringnut 66 kraftschlüssig festgelegt ist, so das die Dichtmembran 43 eine fluidische Trennung zwischen dem Bewegungsraum 59 und einer Umgebung der Betätigungseinrichtung gewährleistet.

In der Fig. 2 ist eine rein schematische Schnittdarstellung einer Membranpumpeneinrichtung 2 gezeigt, die für eine Ankopplung an die Betätigungseinrichtung 1 ausgebildet ist und die für eine Förderung eines Fluids, das an einem Eingangsanschluss 204 bereitgestellt wird, zu einem Ausgangsanschluss 205, genutzt werden kann. Beispielhaft umfasst die Membranpumpeneinrichtung 2 eine quaderförmige Grundplatte 206, die von einer Unterseite 207 mit einer Ausnehmung 208 versehen ist. Die Ausnehmung 208 ist mit einer von der Unterseite 207 montierten Verschlussplatte 210 zumindest weitestgehend ausgefüllt, die in einem der Unterseite 207 abgewandten zentralen Bereich einen Stützvorsprung 211 aufweist, der sich in Richtung der Oberseite 209 erstreckt und der an einer der Unterseite 207 abgewandten Stirnseite 212 eine konkave, insbesondere kugelkalottenförmige Gestalt aufweist. Dadurch wird eine Pumpenkammer 252 in einem Pumpenkammerkörper 253 gebildet, der Teil des Gehäusekörpers 254 der Pumpeneinrichtung 2 ist. Die Ausnehmung 208 in der Grundplatte 206 ist derart ausgebildet, dass nach Montage der Verschlussplatte 210 in die Grundplatte 206 ein nutförmiger Zustromkanal 213 sowie ein nutförmiger Abstromkanal 214 gebildet werden, die jeweils an Mündungsöffnungen 215, 216 benachbart zum Stützvorsprung 211 in Richtung der Oberseite 209 ausmünden. An einem dem Stützvorsprung 211 abgewandten Endbereich mündet der Zustromkanal 213 in einen beispielhaft zylindrisch ausgebildeten Ventilraum 217 eines ersten Membranventils 202, in gleicher Weise mündet der Abstromkanal 214 an einem dem Stützvorsprung 211 abgewandten Endbereich in einen beispielhaft zylindrisch ausgebildeten Ventilraum 218 eines zweiten Membranventils 203.

Das erste Membranventil 202 ist als Einlassventil für eine Beeinflussung eines Zustrompfads 219, der den Eingangsanschluss 204, den Ventilraum 217 und den Zustromkanal 213 umfasst, vorgesehen. Hierzu ist im Ventilraum 217 ein ringförmig ausgebildeter Ventilsitz 220 angeordnet, dessen Stirnseite 221 beabstandet zur Oberseite 209 der Grundplatte 206 angeordnet ist und der zentral von einem Ventilkanal 222 durchsetzt ist. Demgegenüber ist die fluidisch kommunizierende Verbindung zwischen dem Ventilraum 217 und dem Zustromkanal 213 in einem radial außenliegenden Bereich des Ventilraums 217 verwirklicht.

Das zweite Membranventil 203 ist als Auslassventil für eine Beeinflussung eines Abstrompfads 223, der den Ausgangsanschluss 205, den Ventilraum 218 und den Abstromkanal 214 umfasst, vorgesehen. Hierzu ist im Ventilraum 218 ein ringförmig ausgebildeter Ventilsitz 224 angeordnet, der zentral von einem Ventilkanal 240 durchsetzt ist. Die Stirnseite des Ventilsitzes 224 ist beabstandet zur Oberseite 209 der Grundplatte 206 angeordnet. Der Ventilsitz 224 wird von dem Ventilraum konzentrisch umschlossen, wobei der Abstromkanal 214 die Mündungsöffnung 216 der Membranpumpe 200, d. h. deren Auslassöffnung, mit dem Ventilraum 218 verbindet. Die fluidisch kommunizierende Verbindung zwischen dem Ventilraum 218 und dem Ausgangsanschluss 205 ist in einem radial außenliegenden Bereich des Ventilraums 218 verwirklicht. Der Abstrompfad 223 umfasst neben dem zu dem Auslassventil 203 führenden ersten Abstromkanal 214 einen zweiten von dem Auslassventil 203 abgehenden Abstromkanal 214A, der an den Ventilkanal 240 angeschlossen ist. Der zweite Abstromkanal 214A verbindet den Ventilkanal 240 mit dem Ausgangsanschluss 205.

Rein exemplarisch ist die gesamte Oberseite 209 der Grundplatte 206 mit einer gummielastischen Membran 201 überzogen, die beispielsweise stoffschlüssig an der Oberseite 209 festgelegt ist und somit eine fluidische Trennung zwischen den Ventilräumen 217, 218 und einer Umgebung der Membranpumpeneinrichtung 2 gewährleistet. Ferner dient die Membran 201 auch zur Abtrennung eines Pumpraums 227, der von der Ausnehmung 208 sowie vom Stützvorsprung 211 und der Membran 201 berandet wird, von der Umgebung der Membranpumpeneinrichtung 2.

Bei einer Anbringung der Membranpumpeneinrichtung 2 an der Betätigungseinrichtung 1 kann mit Hilfe der in der Fig. 1 dargestellten ersten Stelleinrichtung 9 beispielsweise eine Deformation der Membran 201 über dem Ventilsitz 220 vorgenommen werden, um dadurch die Membran 201 lokal abdichtend auf die Stirnseite 221 des Ventilsitzes 220 zu pressen und damit eine fluidisch kommunizierende Verbindung zwischen dem Eingangsanschluss 204 und dem Zustromkanal 213 zu unterbrechen. Ferner kann mit Hilfe einer spiegelbildlich zu der in Fig. 1 dargestellten zweiten Stelleinrichtung 9 (14), die aus Gründen der Übersichtlichkeit in der Fig. 1 nicht gezeigt ist, eine Deformation der Membran 201 über dem Ventilsitz 224 vorgenommen werden, um dadurch die Membran 201 lokal abdichtend auf die Stirnseite 225 des Ventilsitzes 224 zu pressen und damit eine fluidisch kommunizierende Verbindung zwischen dem ersten Ab stromkanal 214 und dem Ausgangsanschluss 205 zu unterbrechen.

Darüber hinaus kann die Membran 201 im Bereich des Pumpraums 227 oberhalb des Stützvorsprungs 211 durch geeignete Beaufschlagung der Ausnehmung 30 mit Steuerfluid oder einem Unterdruck aus der in Fig. 2 dargestellten Neutralstellung in eine rein schematisch in der Fig. 3 dargestellte konvexe Ansaugkonfiguration oder in eine rein schematisch in der Fig. 3 dargestellte konkave Austragkonfiguration gebracht werden.

Die in der Fig. 3 dargestellte Betätigungseinrichtung 1 ist, wie bereits obenstehend ausgeführt wurde, rein exemplarisch in den Grundkörper 4 und die Steuerbaugruppe 6 aufgeteilt, wobei die im Grundkörper 4 enthaltenen Komponenten bereits im Zusammenhang mit der Fig. 1 vorstehend umfassend beschrieben wurden.

Die Steuerbaugruppe 6 enthält eine Steuereinrichtung 70, die beispielsweise als Mikroprozessor oder Mikrocontroller ausgebildet sein kann und die zur Abarbeitung eines vorgebbaren Ablaufprogramms ausgebildet ist. Exemplarisch ist vorgesehen, dass die Steuereinrichtung 70 über eine Schnittstelle 71 in elektrische Verbindung mit einer nicht dargestellten übergeordneten Steuerungseinrichtung, bei der es sich beispielsweise um eine speicherprogrammierbare Steuerung handeln kann, gebracht werden kann, so dass die übergeordnete Steuerungseinrichtung Steuerbefehl an die Steuereinrichtung 70 bereitstellen kann. Alternativ ist vorgesehen, dass die Steuereinrichtung 70 ohne externe Befehle autark arbeiten kann und die nachstehend näher beschriebenen Komponenten in geeigneter Weise ansteuert. Bei diesen Komponenten handelt es sich zum einen um eine Steuerfluidanordnung 72 zur Bereitstellung von Steuerfluid an die Steuerkammer 8 und zum anderen um eine Arbeitsfluidanordnung 73 zur Bereitstellung von Arbeitsfluid an die beiden schematisch eingezeichneten Stelleinrichtungen 9, 14, die als Einlass-Stelleinrichtung 9 und Auslass-Stelleinrichtung 14 bezeichnet werden.

Die Steuerfluidanordnung 72 umfasst ein erstes Steuerfluidventil 74 und ein zweites Steuerfluidventil 75, die beispielhaft jeweils als Magnetventile ausgebildet sein können und die elektrisch über Steuerleitungen 76, 77 mit der Steuereinrichtung 70 verbunden sind. Das erste Steuerfluidventil 74 steht über die erste Steuerfluidleitung 33, die in der Steuerkammer 8 mit einer ersten Steuerfluidmündung 34 ausmündet, in fluidisch kommunizierender Verbindung. Ferner ist das erste Steuerfluidventil 74 fluidisch kommunizierend mit einem ersten Steuerfluidanschluss 78 verbunden und ermöglicht eine wahlweise Freigabe oder Blockierung einer fluidisch kommunizierenden Verbindung zwischen dem ersten Steuerfluidanschluss 78 und der ersten Steuerfluidmündung 34.

Das zweite Steuerfluidventil 75 steht über eine zweite Steuerfluidleitung 35, die in der Steuerkammer 8 mit einer zweiten Steuerfluidmündung 36 ausmündet, in fluidisch kommunizierender Verbindung. Ferner ist das zweite Steuerfluidventil 75 fluidisch kommunizierend mit einem zweiten Steuerfluidanschluss 79 verbunden und ermöglicht eine wahlweise Freigabe oder Blockierung einer fluidisch kommunizierenden Verbindung zwischen dem zweiten Steuerfluidanschluss 79 und der zweiten Steuerfluidmündung 36.

Vorzugsweise ist vorgesehen, dass am ersten Steuerfluidanschluss 78 eine nicht dargestellte Druckluftquelle angeschlossen ist, während am zweiten Steuerfluidanschluss 79 eine nicht dargestellte Unterdruckquelle angeschlossen ist. Somit kann bei einer Anbringung der in Fig. 2 dargestellten Membranpumpeneinrichtung 2 an der in Fig. 3 dargestellten Betätigungseinrichtung 1 durch geeignete, vorzugsweise wechselweise, insbesondere zyklisch wiederkehrende, Ansteuerung der beiden Steuerfluidventile 74 und 75 eine Abfolge einer Überdruckbeaufschlagung und einer Unterdruckbeaufschlagung für die Steuerkammer 8 bewirkt werden, wodurch die Membran 201 der Membranpumpe 200 aus der Neutralstellung 180 zunächst in eine konvex gewölbte Ansaugstellung 181 und anschließend in eine konkav gewölbte Austragstellung 182 gebracht werden kann. Durch diese wechselnde Deformation der Membran 201 der Membranpumpe 200 wird ein Förderhub für ein in der Membranpumpe 200 aufgenommenes Fluid bewirkt, so dass dieses bei geeigneter Ansteuerung der Membranventile 202 und 203 vom Eingangsanschluss 204 zum Ausgangsanschluss 200 gefördert werden kann. Um diese Ansteuerung der beiden Membranventile 202 und 203 bewirken zu können, sind die Einlass- und Auslass-Stelleinrichtungen 9 und 14 vorgesehen, die jeweils mit den zugeordneten Einlass- und Auslass-Stellgliedern 38 auf die gegenüberliegenden Abschnitte der Membran 201 einwirken können, um wahlweise eine abdichtende Anlage der Membran 201 am jeweiligen Ventilsitz 220, 224 oder eine Freigabe des jeweiligen Ventilsitzes 220, 224 zu ermöglichen.

Zu diesem Zweck umfasst die Einlass-Stelleinrichtung 9, die rein exemplarisch für eine Beeinflussung des dem Zustromkanal 213 zugeordneten Membranventils 202 vorgesehen ist, ein erstes Arbeitsfluidventil 81, das über eine Steuerleitung 83 mit der Steuereinrichtung 70 verbunden ist, um eine elektrische Ansteuerung des Arbeitsfluidventils 81 zu ermöglichen. Ferner ist das Arbeitsfluidventil 81 fluidisch mit dem ersten Steuerfluidanschluss 78 verbunden und über eine erste Arbeitsfluidleitung 85 mit der ersten Stelleinrichtung 9 fluidisch kommunizierend gekoppelt. Dementsprechend kann das Arbeitsfluidventil 81, bei dem es sich insbesondere um ein 3/2-Wege-Magnetventil handeln kann, wahlweise eine Versorgung der Einlass-Stelleinrichtung 9 mit druckbeaufschlagtem Arbeitsfluid oder eine Entlüftung der Stelleinrichtung 9 bewirken, wobei die Entlüftung über einen ersten Entlüftungskanal 87 bis zu einem ersten Schalldämpfer 89 erfolgt.

Wenn sich die Dichtmembran 43 in einer Neutralstellung befindet, in der die Dichtmembran nach unten ausgelenkt ist, presst die Dichtmembran die darunter anbringbare Membran 201 der Membranpumpeneinrichtung 2 auf den Ventilsitz 220, wodurch der Zustromkanal 213 geschlossen wird. Wie aus der Darstellung der Fig. 1 hervorgeht, führt eine Beaufschlagung der ersten Stelleinrichtung 9 mit druckbeaufschlagtem Fluid in dem Arbeitsraum 58 zu einer Stellbewegung des Stößels 39, wodurch die Dichtmembran 43 aus der Neutralstellung in eine Funktionsstellung gebracht wird, in der die Dichtmembran 43 mit dem Gehäuse zumindest nahezu eben ist, so dass die Membran 201 der Membranpumpeneinrichtung 2 aufgrund ihrer Elastizitätseigenschaften von dem Ventilsitz 220 abgehoben wird, so dass der Zustromkanal 213 freigegeben wird. Die Auslass-Stelleinrichtung 14, die rein exemplarisch für eine Beeinflussung des dem Abstromkanal 214 zugeordneten Membranventils 203 vorgesehen ist, umfasst ein zweites Arbeitsfluidventil 82, das über eine Steuerleitung 84 mit der Steuereinrichtung 70 verbunden ist, um eine elektrische Ansteuerung des Arbeitsfluidventils 82 zu ermöglichen. Ferner ist das Arbeitsfluidventil 82 fluidisch mit dem ersten Steuerfluidanschluss 78 verbunden und über eine zweite Arbeitsfluidleitung 86 mit der Auslass-Stelleinrichtung 14 fluidisch kommunizierend gekoppelt. Dementsprechend kann das Arbeitsfluidventil 82, bei dem es sich insbesondere um ein 3/3-Wege-Piezo- Druckregelventil handeln kann, wahlweise eine Versorgung der zweiten Stelleinrichtung 14 mit druckbeaufschlagtem Arbeitsfluid oder eine Entlüftung der zweiten Stelleinrichtung 14 bewirken, wobei die Entlüftung über einen zweiten Entlüftungskanal 88 bis zu einem zweiten Schalldämpfer 90 erfolgt.

Wie aus der Darstellung der Fig. 1 hervorgeht, führt eine Beaufschlagung der Auslass-Stelleinrichtung 14 mit druckbeaufschlagtem Fluid zu einer Stellbewegung des Stößels 39, wodurch die in der Fig. 1 in einer Neutralstellung nach unten ausgelenkte Dichtmembran 43, die die darunter anbringbare Membran 201 der Membranpumpeneinrichtung 2 auf den Ventilsitz 224 presst, wodurch eine Blockierwirkung für den Abstromkanal 214 hervorgerufen wird, in eine Funktionsstellung gebracht werden kann, in der die Dichtmembran 43 zumindest nahezu eben ist, wie dies in der Fig. 1 dargestellt ist und wodurch die darunter anbringbare Membran 201 der Membranpumpeneinrichtung 2 aufgrund ihrer Elastizitätseigenschaften vom Ventilsitz 224 abgehoben wird und den Abstromkanal 214 freigibt.

Für eine Druckregelung in der Steuerkammer 8 ist ein Drucksensor 91 vorgesehen, der über eine Sensorleitung 92 elektrisch mit der Steuereinrichtung 70 verbunden ist und der rein exemplarisch mittels einer Fluidleitung 94 in fluidisch kommunizierender Verbindung mit der ersten Steuerfluidleitung 33 steht. Hierdurch kann der Drucksensor 91 den in der Steuerkammer 8 vorliegenden Fluiddruck ermitteln und als elektrisches Signal über die Sensorleitung 92 an die Steuereinrichtung 70 weiterleiten.

Beispielhaft ist zusätzlich dem zweiten Arbeitsfluidventil 82 ein Drucksensor 93 zugeordnet, der rein exemplarisch in das zweite Arbeitsfluidventil 82 integriert ist und über die zugeordnete zweite Steuerleitung 84 elektrisch mit der Steuereinrichtung 70 verbunden ist.

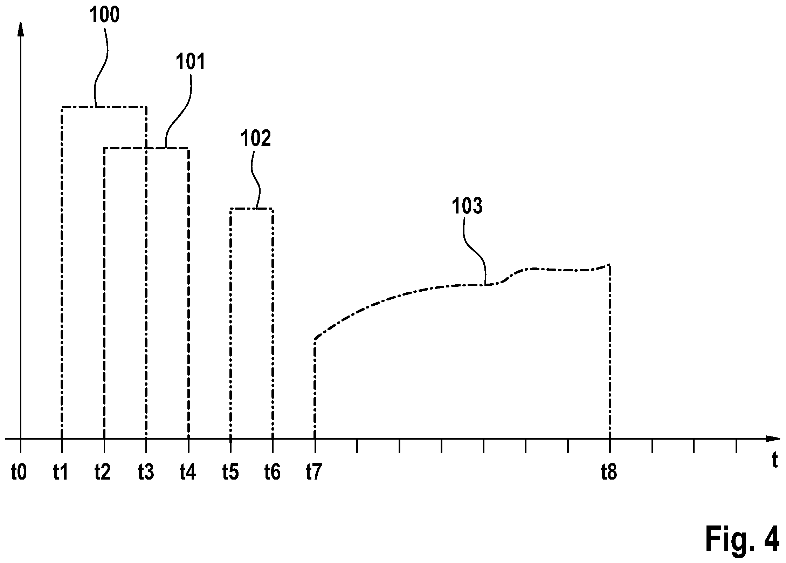

In der Fig. 4 ist ein streng schematischer, modellhafter Ablauf für die Ansteuersignale 101 bis 104 dargestellt, die zur Ansteuerung der einzelnen Komponenten der Betätigungseinrichtung 1 von der Steuereinrichtung 70 bereitgestellt werden. Dabei sind weder die Signalpegel noch die Zeitabschnitte maßstäblich gewählt.

Zu einem Zeitpunkt t0 wird von der Steuereinrichtung 70 kein Ansteuersignal ausgegeben. Somit befinden sich die beiden Stelleinrichtungen 9, 14 jeweils in einer Ruhestellung, in der das zugeordnete Stellglied 38 die jeweilige Dichtmembran 43 jeweils von der Montagefläche 7 abragen lässt und somit eine Deformation der Membran der jeweils gegenüberliegenden Membranventile 202, 203 gewährleistet ist. Dies geht aus der Fig. 3 anhand der strichpunktierten Membran der Membranventile 202, 203 hervor. Somit sind zu diesem Zeitpunkt sowohl der Zustromkanal 213 als auch der Abstromkanal 214 blockiert.

Zu einem Zeitpunkt t1 gibt die Steuereinrichtung 70 ein Ansteuersignal 100 für das erste Arbeitsfluidventil 81 aus, wodurch dieses aus einer Entlüftungsstellung in eine Belüftungsstellung geschaltet wird und eine Druckbeaufschlagung der Einlass-Stelleinrichtung 9 erfolgt. Aufgrund dieser Druckbeaufschlagung bewegt sich der Stößel 39 der Einlass-Stelleinrichtung 9 aus der Neutralstellung gemäß der Fig. 3, in der die Dichtmembran 43 von der Montagefläche 7 abragt und die in Fig. 3 strichtpunktiert dargestellte Membran des zugeordneten Membranventils 202 deformiert wird, in eine Funktionsstellung, wie sie in der Fig. 3 gestrichelt dargestellt ist. Dadurch wird das gegenüberliegend anbringbare Membranventil 202 der Membranpumpeneinrichtung 2 geöffnet, wie dies in der Fig. 3 gestrichelt dargestellt ist, und ermöglicht zumindest prinzipiell einen Zustrom von Fluid vom Eingangsanschluss 204 in die Membranpumpe 200.

Zu einem Zeitpunkt t2 gibt die Steuerschaltung 70 ein Ansteuersignal 101 an das zweite Steuerfluidventil 75 aus, wodurch dieses aus einer Blockierstellung in eine Freigabestellung geschaltet wird und eine Unterdruckbeaufschlagung der Steuerkammer 8 erfolgt. Durch diese Unterdruckbeaufschlagung wird die Membran 201 der Membranpumpe 200 in die Steuerkammer 8 gesaugt und legt sich an der Anlagefläche 31 des Formkörpers 26 an, so dass sie die konvex gewölbte Ansaugstellung 181 einnimmt. Zu diesem Zeitpunkt weist die Pumpkammer 227 der Membranpumpe 200 ein maximales Volumen auf, wobei im Zuge der Deformation der Membran 201 das zu fördernde Fluid vom Eingangsanschluss 204 in die Pumpkammer 227 gesaugt wird.

Zu einem Zeitpunkt t3 schaltet die Steuerschaltung 70 das Ansteuersignal 100 für das erste Arbeitsfluidventil 81 ab, wodurch dieses aus der Belüftungsstellung in die Entlüftungsstellung geschaltet wird und eine Druckbeaufschlagung der Einlass-Stelleinrichtung 9 beendet wird. Dadurch bewegt sich der Stößel 39 der Einlass-Stelleinrichtung 9, insbesondere aufgrund einer Federwirkung einer nicht dargestellten Rückstellfeder, aus der in Fig. 1 dargestellten Funktionsstellung in eine von der Montagefläche 7 abragende Neutralstellung, wie sie in der Fig. 3 strichpunktiert dargestellt ist, so dass das gegenüberliegend anbringbare Membranventil 202 der Membranpumpeneinrichtung 2 geschlossen wird. Zu diesem Zeitpunkt t3 ist der Zustromkanal 213 in der Membranpumpeneinrichtung 2 blockiert und es wird ein Entweichen von Fluid aus der Pumpkammer 227 der Membranpumpe 200 verhindert.

Zu einem Zeitpunkt t4 wird das Ansteuersignal 101 durch die Steuerschaltung 70 abgeschaltet, so dass das zweite Steuerfluidventil 75 wieder die Blockierstellung einnimmt und die Steuerkammer 8 ab diesem Zeitpunkt t4 zunächst einen konstanten Unterdruck aufweist.

Zu einem Zeitpunkt t5 gibt die Steuerschaltung 70 ein Ansteuersignal 102 an das erste Steuerfluidventil 74 aus, wodurch dieses aus einer Blockierstellung in eine Freigabestellung geschaltet wird und eine Druckbeaufschlagung der Steuerkammer 8 erfolgt. Durch diese Druckbeaufschlagung wird das in der Membranpumpe 200 aufgenommene Fluid zusätzlich unter Druck gesetzt. Vorzugsweise ist der Druck in der Steuerkammer 8 so bemessen, dass bei einem nachfolgenden Austragvorgang eine vollständige Verformung der Membran 201 der Membranpumpe 200 aus der konvex gewölbten Ansaugstellung 181 in die konkav gewölbte Austragstellung erfolgt, so dass mit Hilfe der Membranpumpe 200 eine maximale Fluidmenge gefördert werden kann.

Zu einem Zeitpunkt t6 wird das Ansteuersignal 102 durch die Steuerschaltung 70 abgeschaltet, so dass das erste Steuerfluidventil 74 wieder die Blockierstellung einnimmt.

Zu einem Zeitpunkt t7 stellt die Steuerschaltung 70 ein Ansteuersignal 103 für das zweite Arbeitsfluidventil 82 bereit, damit dieses eine geregelte Stellbewegung des der Auslass-Stelleinrichtung 14 zugehörigen Stößels 39 durchführen kann. Dabei wird das Ansteuersignal 103 in Abhängigkeit von Drucksignalen des Drucksensors 91 in der Steuerschaltung 70 unter Zuhilfenahme eines Beobachteralgorithmus berechnet, um beispielsweise einen möglichst konstanten Fluidmassenstrom aus der Membranpumpe 200 zum Ausgangsanschluss 205 zu bewirken.

Dementsprechend verändert sich die Stellung des Stößels 39 dynamisch während des Fluidfördervorgangs, wobei das Membranventil 203 in der Art eines Proportionalventils betrieben wird. Aufgrund des Überdrucks in der Steuerkammer 8 wird das Fluid in der Pumpkammer 227 der Membranpumpe 200 zum Ausgangsanschluss 205 gedrängt, dieser Vorgang hält vorzugsweise solange an, bis die Membran 201 flächig an der Stirnseite 212 des Stützvorsprungs 211 anliegt und somit die Pumpkammer 227 zumindest nahezu vollständig entleert ist.

Zu einem Zeitpunkt t8 schaltet die Steuerschaltung 70 das Ansteuersignal 103 ab, so dass zu diesem Zeitpunkt der Fördervorgang für die Membranpumpe 200 beendet ist und ein neuer Fördervorgang beginnen kann.

Die Fig. 5 zeigt eine schematische Darstellung des erfindungsgemäßen Auslassventils 203, wobei die einzelnen Komponenten des Ventils 203 mit denselben Bezugszeichen wie in den Figuren 1 bis 4 bezeichnet sind. Diese Auslassventilanordnung mit den Zu- und Ableitungen wird als Ventilanordnung mit einer "mechanischen Gegenkopplung" bezeichnet.

In der schematischen Darstellung wird die Auslass-Stelleinrichtung 14 mit dem Auslassstellglied 38 durch einen mit einer Feder 250 vorgespannten Stößel 251 dargestellt, der auf die elastische Membran 201 des Auslassventils 203 eine Anpresskraft F ausübt. Der Stößel übt auf die Membran eine Kraft F aus, die der Differenz der Druckkraft F_{F} der Feder und einer Kraft F_{S} entspricht, die von der Auslass-Stelleinrichtung 14 entgegen der Federkraft auf den Stößel 251 ausgeübt werden kann, um den Stößel von dem Auslassventilsitz 225 abzuheben. Die Feder 250 hält den Stößel 251 in der Ruhestellung, in der das Auslassventil 203 geschlossen ist, d. h. der Stößel drückt die Membran 201 auf den Ventilsitz 225. In Fig. 5 ist das Auslassventil 203 in der Ruhestellung dargestellt. In der Funktionsstellung drückt der Stößel die Membran nicht auf den Ventilsitz, so dass das Auslassventil geöffnet ist.

Der Ventilraum 218 des Auslassventils 203 ist in einem Ventilkörper 254A ausgebildet, der Teil des Gehäusekörpers 254 der Pumpeneinrichtung 2 ist. Der Abstrompfad 223 umfasst einen ersten Abstromkanal 204, der von der Pumpenkammer 252 zu dem Ventilraum 218 des Auslassventils 203 führt, und einen zweiten Abstromkanal 204A, der von dem Auslassventilkanal 240 zu dem Ausgangsanschluss 205 führt. Die Querschnittsfläche des Auslassventilsitzes 225 und des Auslassventilkanals 240 ist kleiner als die Querschnittsfläche des den Auslassventilsitz 225 umschließenden Bereichs des Auslassventilraums 218. Folglich ist die äußere Fläche A₁ der Membran 201, die den Ventilsitz umschließt, größer als die innere Fläche A₂ der Membran 201, die auf dem Ventilsitz 225 aufliegt, was in Fig. 5 veranschaulicht ist.

Zu Beginn des Pumpenschlags steht das Fluid in der Pumpenkammer 252 unter Druck. Da die Pumpenkammer 252 über den ersten Abstromkanal 204A mit dem Ventilraum 225 des Auslassventils 203 in Fluidverbindung steht, wird die äußere Fläche A₁ der Membran 201, die den Ventilsitz 225 umschließt, von dem Fluid mit Druck beaufschlagt. Auf die äußere Fläche A₁ der Membran 201 wirkt daher eine Kraft F₁, die dem Produkt von dem Druck p des Fluids und der äußeren Fläche A₁ der Membran 201 entspricht. Die innere Fläche A₂ der Membran 201 wird hingegen nicht mit Druck beaufschlagt, da die Membran mit der inneren Fläche noch auf dem Ventilsitz aufliegt, was der Ruhestellung des Auslassventils entspricht.

Während des Pumpenschlags wird der Stößel 251 entgegen der Anpresskraft der Feder 250 von dem zuströmenden Fluid angehoben, wobei nach dem Öffnen des Auslassventils 203 der Druck p des zuströmenden Fluids abnimmt. Zu Beginn des Pumpenschlags muss die Federkraft des Stößels 251, der die Membran 201 auf den Ventilsitz 225 drückt, zunächst überwunden werden. Diese Kraft wird dadurch überwunden, dass auf den Stößel von dem Fluid eine Kraft ausgeübt wird, die dem Produkt von dem Druck p des Fluids und der äußeren Fläche A₁ der Membran entspricht. Sobald die Membran von dem Ventilsitz abgehoben ist, wird auch die innere Fläche A₂ der Membran 201 von dem Fluid mit Druck beaufschlagt. Da die innere Fläche A₂ der Membran aber kleiner als die äußere Fläche A₁ ist, nimmt die Kraft, mit der die Membran von dem Ventilsitz gedrückt wird, aufgrund der relativ geringen Änderung der wirksamen Fläche nur geringfügig zu, so dass bei abnehmenden Pumpendruck die Rückstellkraft des federnd vorgespannten Stößels schnell wieder überwiegt. Das Auslassventil 203 zeigt daher beim Öffnen ein Verhalten, dass mit fortschreitendem Pumpenschlag eine immer größer werdende Betätigungskraft am Stößel erfordert, um stetig weiter zu fördern.

Fig. 6 zeigt als Vergleichsbeispiel eine andere Auslassventilanordnung, bei der der Abstrompfad 223 einen ersten Abstromkanal 214 umfasst, der die Auslassöffnung der Pumpenkammer 252 mit dem Auslassventilkanal 240 verbindet, und einen zweiten Abstromkanal 214A umfasst, der den Auslassventilraum 218 mit dem Ausgangsanschluss 205 verbindet, wobei die Querschnittsfläche des Auslassventilsitzes 225 kleiner als die Querschnittsfläche des den Ventilsitz umschließenden Bereichs des Ventilraums 218 ist. Dieser Fall wird als "mechanische Mitkopplung" bezeichnet. Bei einer derartigen Anordnung ist beim Abheben der Membran 201 von dem Ventilsitz die Änderung der wirksamen Fläche größer als bei dem Fall der "mechanischen Gegenkopplung". Folglich wirkt der Staudruck beim Abheben der Membran auf eine sehr viel größere Fläche. Aufgrund dieser deutlich größeren Veränderung der Wirkfläche überwiegt die von dem Fluid auf den Stößel ausgeübten Kraft. Das Auslassventil zeigt daher beim Öffnen ein Verhalten, dass mit fortschreitendem Pumpenschlag eine immer kleiner werdende Betätigungskraft am Stößel erfordert, um stetig weiter zu fördern. Dies steht im Widerspruch dazu, dass zunächst eine Kraft aufgebaut werden muss, um den Stößel von dem Ventilsitz abzuheben. Es hat sich gezeigt, dass eine derartige Ventilanordnung zu einem nicht regelbaren Verhalten neigt. In Versuchen haben sich Schwingungen gezeigt.

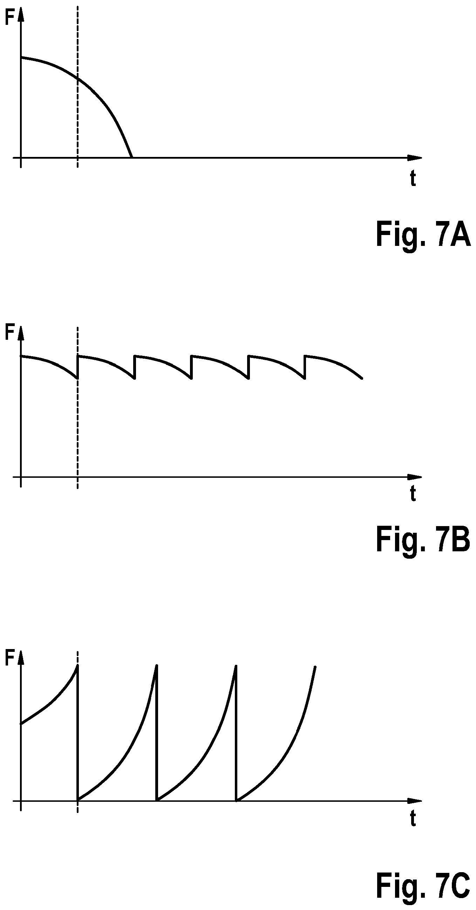

Fig. 7A zeigt bei der erfindungsgemäßen Auslassventilanordnung (Fig. 5) den zeitlichen Verlauf der auf den Stößel 251 in Öffnungsrichtung wirkenden Kraft für den Fall, dass eine Regelung der Flüssigkeitsströmung, wie sie unter Bezugnahme auf die Figuren 1 bis 4 beschrieben ist, nicht erfolgt. Es zeigt sich, dass die Kraft relativ schnell auf Null abfällt. Fig. 7B zeigt bei der erfindungsgemäßen Auslassventilanordnung (Fig. 5) den zeitlichen Verlauf der auf den Stößel in Öffnungsrichtung wirkenden Kraft bei der unter Bezugnahme auf die Figuren 1 bis 4 beschriebenen Regelung der Flüssigkeitsströmung, um eine konstante Flussrate zu gewährleisten, während Fig. 7C die auf den Stößel in Öffnungsrichtung wirkenden Kraft als Funktion der Zeit für den Fall der "mechanischen Mitkopplung" zeigt (Fig. 6). Im Gegensatz zu dem Fall der "mechanischen Mitkopplung" (Fig. 6) zeigt sich für den Fall der "mechanischen Gegenkopplung" (Fig. 5) ein deutlich gleichmäßiger Verlauf. In dem Fall der "mechanischen Mitkopplung" (Fig. 6) zeigt sich ein periodischer Anstieg und Abfall der Kraft zwischen einem Maximalwert und Null.

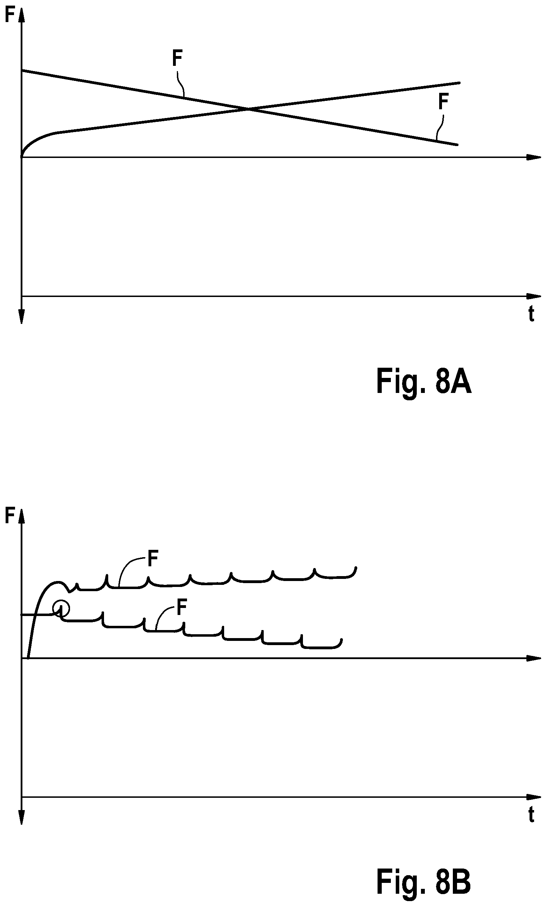

Die oben beschriebenen Unterschiede zwischen einer "mechanischen Gegenkopplung" und einer "mechanischen Mitkopplung" werden unter Bezugnahme auf die Figuren 8A und 8B nochmals erläutert.

Die das Ventil öffnenden Kräfte lassen sich in die beiden Anteile F_{S} (Steuerfluid) und F_{A} (Arbeitsfluid) unterteilen. Ein Durchfluss ergibt sich dann, wenn die Federkraft F_{F} = F_{S} + F_{A} ist. Dies ist der Zustand, in dem keine Anpresskraft auf den Ventilsitz ausgeübt wird.

Fig. 8A zeigt für den Fall der "mechanischen Gegenkopplung" den zeitlichen Verlauf der Kräfte Fp, F_{S}, F_{A}. Bei der "mechanischen Gegenkopplung" nehmen die der Federkraft Fp entgegenwirkenden Kräfte F_{S} bzw. F_{A} zu bzw. ab. Während eines Pumpenschlags steigt die Kraft F_{S} monoton an und die Kraft F_{A} fällt monoton ab. Da die Regelung F_{S} monoton ansteigen lässt, wird ein gleichmäßiger kontinuierlicher Fluss erzeugt.

Fig. 8A zeigt den Fall der "mechanischen Mitkopplung", bei dem sich aufgrund der oben beschriebenen Flächenverhältnisse beim Öffnen des Ventils ein kurzzeitiger Anstieg der Kraft F_{A} zeigt. Dieser Kraftanstieg muss dann durch die Regelung mittels einer Absenkung des Drucks in der Steuerkammer des Antriebs ausgeglichen werden. Der Regler senkt F_{S} soweit ab, dass das Ventil wieder schließt. Der zeitliche Verlauf der Kräfte F_{A} und F_{S} ist während des Pumpenschlags nicht monoton verlaufend. Der Fluss wechselt ständig zwischen "hoher Durchfluss" und "kein Durchfluss".

## Patentansprüche

1. Membranpumpeneinrichtung zur Förderung von Fluiden, insbesondere von medizinischen Flüssigkeiten für eine Blutbehandlung, mit
einem Pumpenkammerkörper (253), in dem eine Ausnehmung ausgebildet ist, die zur Bildung einer Pumpenkammer (252) von einer elastischen Membran (201) verschlossen ist,
einem Zustrompfad (219), der einen Eingangsanschluss (204) mit einer Einlassöffnung (215) der Pumpenkammer (252) verbindet,
einem Abstrompfad (223), der eine Auslassöffnung (216) der Pumpenkammer (252) mit einem Ausgangsanschluss (254) verbindet,
einem in dem Zustrompfad (219) vorgesehenen Einlassventil (202) zur Beeinflussung der Flüssigkeitsströmung in dem Zustrompfad und einem in dem Abstrompfad (223) vorgesehenen Auslassventil (203) zur Beeinflussung der Flüssigkeitsströmung in dem Abstrompfad, wobei das Auslassventil (203) ein Membranventil ist, das einen Auslassventilkörper (254A) aufweist, in dem eine Ausnehmung ausgebildet ist, die zur Bildung eines Auslassventilraums (218) von einer elastischen Membran (201) verschlossen ist, in welchem ein Auslassventilsitz (225) angeordnet ist, dessen Stirnseite der Membran (201) zugewandt und in der Öffnungsstellung des Auslassventils im Abstand zu der Membran angeordnet ist, wobei der Auslassventilsitz (225) von einem Auslassventilkanal (240) durchsetzt ist, und
der Abstrompfad (223) einen ersten Abstromkanal (214) umfasst, der die Auslassöffnung (216) der Pumpenkammer (252) mit dem Auslassventilraum (218) direkt verbindet, und einen zweiten Abstromkanal (214A) umfasst, der den von der elastischen Membran (201) verschließbaren Auslassventilkanal (240) mit dem Ausgangsanschluss (205) verbindet, und **dadurch gekennzeichnet, dass**
die Querschnittsfläche des Auslassventilsitzes (225) kleiner als die Querschnittsfläche des den Auslassventilsitz umschließenden Bereichs des Auslassventilraums (218) ist.

2. Membranpumpeneinrichtung nach Anspruch 1, **dadurch gekennzeichnet, dass** der Auslassventilsitz (225) ein ringförmiger Ventilsitz ist.

3. Membranpumpeneinrichtung nach Anspruch 1 oder 2, **dadurch gekennzeichnet, dass** das Einlassventil (201) ein Membranventil ist, das einen Einlassventilkörper aufweist, in dem eine Ausnehmung ausgebildet ist, die zur Bildung eines Einlassventilraums (217) von einer elastischen Membran (201) verschlossen ist, in welchem ein Einlassventilsitz (221) angeordnet ist, dessen Stirnseite der Membran (201) zugewandt und in der Öffnungsstellung des Einlassventils im Abstand zu der Membran angeordnet ist, wobei der Einlassventilsitz (221) von einem Einlassventilkanal (222) durchsetzt ist, und
der Zustrompfad (219) einen ersten Zustromkanal umfasst, der den Eingangsanschluss (204) mit dem Einlassventilkanal (222) direkt verbindet, und einen zweiten Zustromkanal (213) umfasst, der den von der elastischen Membran (201) verschließbaren Einlassventilraum (217) mit der Einlassöffnung (215) der Pumpenkammer (252) verbindet.

4. Membranpumpeneinrichtung nach Anspruch 3, **dadurch gekennzeichnet, dass** der Einlassventilsitz (221) ein ringförmiger Ventilsitz ist.

5. Membranpumpeneinrichtung nach Anspruch 3 oder 4, **dadurch gekennzeichnet, dass** die Membranpumpeneinrichtung oder ein Teil der Membranpumpeneinrichtung als eine zur einmaligen Verwendung bestimmte Kassette ausgebildet ist, die für die Verwendung mit einer Betätigungseinrichtung (1) einer Membranpumpe bestimmt ist.

6. Membranpumpeneinrichtung nach Anspruch 5, **dadurch gekennzeichnet, dass** der Pumpenkammerkörper (252) und der Einlassventil- und Auslassventilkörper (254A) Bestandteil eines einteiligen oder mehrteiligen Gehäusekörpers (254) der Kassette sind, wobei die Membran (201) der Pumpenkammer und die Membran (201) des Einlass- und Auslassventils (202, 203) an einer Anlagefläche angeordnet sind, die an eine Montagefläche (7) der Betätigungseinrichtung (1) ankoppelbar ist.

7. Membranpumpe mit einer Pumpeneinrichtung (2) nach einem der Ansprüche 3 bis 6 und einer Betätigungseinrichtung (1), die derart ausgebildet ist, dass die Membran (201) der Pumpenkammer (252) zwischen einer Ansaugkonfiguration und einer Austragkonfiguration deformierbar wird, wobei das Einlass- und Auslassventil (202, 203) wechselweise öffenbar und schließbar ist, so dass von der Membranpumpe Fluid förderbar ist.

8. Membranpumpe nach Anspruch 7, **dadurch gekennzeichnet, dass** die Betätigungseinrichtung (1) eine Auslass-Stelleinrichtung (14) aufweist, die ein Auslassstellglied (38) zur Deformation der Membran (201) des Auslassventils (203) aufweist, das in eine Ruheposition federnd vorgespannt ist, in der die Membran des Auslassventils auf dem Auslassventilsitz (225) sitzt, so dass das Auslassventil geschlossen ist, wobei das Auslassstellglied aus der Ruheposition in eine Funktionsstellung bewegbar ist, in der die Membran des Auslassventils im Abstand zu dem Auslassventilsitz angeordnet ist, so dass das Auslassventil geöffnet ist.

9. Membranpumpe nach Anspruch 7 oder 8, **dadurch gekennzeichnet, dass** die Betätigungseinrichtung (1) eine Einlass-Stelleinrichtung (9) aufweist, die ein Einlass-Stellglied (38) zur Deformation der Membran (201) des Einlassventils (202) aufweist, das in eine Ruheposition federnd vorgespannt ist, in der die Membran des Einlassventils auf dem Einlassventilsitz (202) sitzt, so dass das Einlassventil geschlossen ist, wobei das Einlassstellglied aus der Ruheposition in eine Funktionsstellung bewegbar ist, in der die Membran des Einlassventils im Abstand zu dem Einlassventilsitz angeordnet ist, so dass das Einlassventil geöffnet ist.

10. Membranpumpe nach Anspruch 9, **dadurch gekennzeichnet, dass** die Betätigungseinrichtung (1) eine erste Arbeitsfluidleitung zur Versorgung der Einlass-Stelleinrichtung (9) mit einem Arbeitsfluid zur Betätigung des Einlassstellgliedes (38) und eine zweite Arbeitsfluidleitung zur Versorgung der Auslass-Stelleinrichtung (14) mit einem Arbeitsfluid zur Betätigung des Auslassstellgliedes (38) aufweist, und dass die Einlass-Stelleinrichtung (9) ein erstes Arbeitsfluidventil (81) zur Beeinflussung eines Querschnitts der ersten Arbeitsfluidleitung und die Auslass-Stelleinrichtung (14) ein zweites Arbeitsfluidventil (82) zur Beeinflussung eines Querschnitts der zweiten Arbeitsfluidleitung aufweist, wobei das erste Arbeitsfluidventil (81) als Schaltventil und das zweite Arbeitsfluidventil (82) als Proportionalventil ausgebildet sind.

11. Membranpumpe nach Anspruch 10, **dadurch gekennzeichnet, dass** die Betätigungseinrichtung (1) eine Steuereinrichtung (70) aufweist, mit der das erste und zweite Arbeitsfluidventil (81, 82) elektrisch verbunden sind, wobei die Steuereinrichtung (70) derart konfiguriert ist, dass in Abhängigkeit von dem Fluidmassenstrom der Membranpumpeneinrichtung (2) ein Drucksollwert für das zweite Arbeitsfluidventil (82) derart bestimmt wird, dass der Fluidmassenstrom während der Austragsphase der Membranpumpeneinrichtung konstant ist.

12. Membranpumpe nach einem der Ansprüche 7 bis 11, **dadurch gekennzeichnet, dass** die Betätigungseinrichtung (1) eine Steuerkammer (8) und eine Steuerfluidleitung zur Beaufschlagung der Steuerkammer mit einem Steuerfluid aufweist, in welcher ein Steuerfluidventil (74, 75) angeordnet ist, das zur Veränderung eines Querschnitts der Steuerfluidleitung ausgebildet ist, wobei die Membran (201) der Pumpenkammer (252) durch Beaufschlagung mit dem Steuerfluid in eine Ansaugkonfiguration oder eine Austragkonfiguration deformierbar ist.

13. Membranpumpe nach Anspruch 12, **dadurch gekennzeichnet, dass** ein Steuerfluid-Drucksensor (91) vorgesehen ist, der elektrisch mit der Steuereinrichtung (70) verbunden ist, wobei die Steuereinrichtung (70) derart konfiguriert ist, dass der Fluidmassenstrom in Abhängigkeit von dem Drucksignal des Steuerfluid-Drucksensors (91) erzeugt wird.

14. Blutbehandlungsvorrichtung, insbesondere Dialysevorrichtung, mit einem Behältnis zur Bereitstellung einer medizinischen Flüssigkeit, insbesondere einer Antikoagulations-Lösung, und einer Membranpumpe nach einem der Ansprüche 7 bis 13 zum Fördern der medizinischen Flüssigkeit.

## Claims

1. Membrane pump device for conveying fluids, in particular medical liquids for blood treatment, with
a pump chamber body (253) in which a recess which is closed by an elastic membrane (201) to form a pump chamber (252) is constructed,
an inward flow path (219) which connects an entry connection (204) to an inlet opening (215) of the pump chamber (252),
an outward flow path (223) which connects an outlet opening (216) of the pump chamber (252) to an exit connection (254),
an inlet valve (202) provided in the inward flow path (219) for influencing the flow of liquid in the inward flow path and an outlet valve (203) provided in the outward flow path (223) for influencing the flow of liquid in the outward flow path,
the outlet valve (203) being a membrane valve which has an outlet valve body (254A) in which a recess is constructed which is closed by an elastic membrane (201) to form an outlet valve chamber (218) in which an outlet valve seat (225) is arranged, the front face of which faces the membrane (201) and in the open position of the outlet valve is arranged at a distance from the membrane, an outlet valve channel (240) passing through the outlet valve seat (225), and
the outward flow path (223) comprising a first outward flow channel (214) which directly connects the outlet opening (216) of the pump chamber (252) to the outlet valve chamber (218), and comprising a second outward flow channel (214A) which connects the outlet valve channel (240), which is closable by the elastic membrane (201), to the exit connection (205), and
**characterized in that**
the cross-section area of the outlet valve seat (225) is smaller than the cross-section area of the region of the outlet valve chamber (218) surrounding the outlet valve seat.

2. Membrane pump device according to claim 1, **characterised in that** the outlet valve seat (225) is an annular valve seat.

3. Membrane pump device according to either of claim 1 or claim 2, **characterised in that** the inlet valve (201) is a membrane valve which has an inlet valve body in which a recess is constructed which is closed by an elastic membrane (201) to form an inlet valve chamber (217) in which an inlet valve seat is arranged (221), the front face of which faces the membrane (201) and in the open position of the inlet valve is arranged at a distance from the membrane, wherein an inlet valve channel (222) passes through the inlet valve seat (221), and
the inward flow path (219) comprises a first inward flow channel which directly connects the entry connection (204) to the inlet valve channel (222), and comprises a second inward flow channel (213) which connects the inlet valve chamber (217), which is closable by the elastic membrane (201), to the inlet opening (215) of the pump chamber (252).

4. Membrane pump device according to claim 3, **characterised in that** the inlet valve seat (221) is an annular valve seat.

5. Membrane pump device according to either claim 3 or claim 4, **characterised in that** the membrane pump device or a part of the membrane pump device is constructed as a cassette intended for a single use, which is intended for use with an actuating device (1) of a membrane pump.

6. Membrane pump device according to claim 5, **characterised in that** the pump chamber body (252) and the inlet valve and outlet valve body (254A) are a constituent of a one-component or multi-component housing body (254) of the cassette, wherein the membrane (201) of the pump chamber and the membrane (201) of the inlet and outlet valve (202, 203) are arranged on a mounting surface which can be coupled to an assembly surface (7) of the actuating device (1).

7. Membrane pump with a pump device (2) according to any of claims 3 to 6 and an actuating device (1) which is constructed such that the membrane (201) of the pump chamber (252) becomes deformable between a suction configuration and a discharge configuration, wherein the inlet and outlet valve (202, 203) can be alternately opened and closed so that fluid can be conveyed by the membrane pump.

8. Membrane pump according to claim 7, **characterised in that** the actuating device (1) has an outlet adjusting device (14) which has an outlet control element (38) for deforming the membrane (201) of the outlet valve (203), which is spring-loaded in a rest position in which the membrane of the outlet valve sits on the outlet valve seat (225), so that the outlet valve is closed, wherein the outlet control element can be moved out of the rest position into a functioning position in which the membrane of the outlet valve is arranged at a distance from the outlet valve seat so that the outlet valve is opened.

9. Membrane pump according to either claim 7 or claim 8, **characterised in that** the actuating device (1) has an inlet adjusting device (9) which has an inlet control element (38) for deforming the membrane (201) of the inlet valve (202), which is spring-loaded in a rest position in which the membrane of the inlet valve sits on the inlet valve seat (202), so that the inlet valve is closed, wherein the inlet control element can be moved out of the rest position into a functioning position in which the membrane of the inlet valve is arranged at a distance from the inlet valve seat so that the inlet valve is opened.

10. Membrane pump according to claim 9, **characterised in that** the actuating device (1) has a first working fluid line for supplying the inlet adjusting device (9) with a working fluid for actuating the inlet control element (38) and a second working fluid line for supplying the outlet adjusting device (14) with a working fluid for actuating the outlet control element (38), and **in that** the inlet adjusting device (9) has a first working fluid valve (81) for influencing a cross-section of the first working fluid line and the outlet adjusting device (14) has a second working fluid valve (82) for influencing a cross-section of the second working fluid line, wherein the first working fluid valve (81) is constructed as a switching valve and the second working fluid valve (82) is constructed as a proportional valve.

11. Membrane pump according to claim 10, **characterised in that** the actuating device (1) has a control device (70), with which the first and second working fluid valve (81, 82) are connected electrically, wherein the control device (70) is configured such that an intended pressure value for the second working fluid valve (82) is determined as a function of the fluid mass flow of the membrane pump device (2) such that the fluid mass flow is constant during the discharge phase of the membrane pump device.

12. Membrane pump according to any of claims 7 to 11, **characterised in that** the actuating device (1) has a control chamber (8) and a control fluid line for charging the control chamber with a control fluid, in which a control fluid valve (74, 75), which is constructed for changing a cross-section of the control fluid line, is arranged, wherein the membrane (201) of the pump chamber (252) is deformable into a suction configuration or a discharge configuration by charging with the control fluid.

13. Membrane pump according to claim 12, **characterised in that** a control fluid pressure sensor (91) which is connected electrically to the control device (70) is provided, wherein the control device (70) is configured such that the fluid mass flow is generated as a function of the pressure signal of the control fluid pressure sensor (91).

14. Blood treatment apparatus, in particular dialysis apparatus, with a container for providing a medical liquid, in particular an anticoagulation solution, and a membrane pump according to any of claims 7 to 13 for conveying the medical liquid.

## Revendications

1. Dispositif de pompe à membrane pour le refoulement de fluides, en particulier de liquides médicaux pour un traitement sanguin, avec
un corps de chambre de pompe (253) dans lequel un évidement est réalisé, lequel est fermé pour la formation d'une chambre de pompe (252) par une membrane élastique (201),
un chemin d'afflux (219) qui relie un raccord d'entrée (204) à une ouverture d'entrée (215) de la chambre de pompe (252),
un chemin d'évacuation (223) qui relie une ouverture de sortie (216) de la chambre de pompe (252) à un raccord de sortie (254),
une soupape d'entrée (202) prévue dans le chemin d'afflux (219) pour la gestion de l'écoulement de liquide dans le chemin d'afflux et une soupape de sortie (203) prévue dans le chemin d'évacuation (223) pour la gestion de l'écoulement de liquide dans le chemin d'évacuation,
dans lequel
la soupape de sortie (203) est une soupape de membrane qui présente un corps de soupape de sortie (254A) dans lequel un évidement est réalisé, lequel est fermé pour la formation d'un espace de soupape de sortie (218) par une membrane élastique (201), dans laquelle un siège de soupape de sortie (225) est agencé, dont le côté avant est tourné vers la membrane (201) et est agencé dans la position d'ouverture de la soupape de sortie à distance de la membrane, dans lequel le siège de soupape de sortie (225) est traversé par un canal de soupape de sortie (240), et
le chemin d'évacuation (223) comporte un premier canal d'évacuation (214) qui relie directement l'ouverture de sortie (216) de la chambre de pompe (252) à l'espace de soupape de sortie (218), et un second canal d'évacuation (214A) qui relie le canal de soupape de sortie (240) refermable par la membrane élastique (201) au raccord de sortie (205), et
**caractérisé en ce que**
la superficie du siège de soupape de sortie (225) est inférieure à la superficie de la zone entourant le siège de soupape de sortie de l'espace de soupape de sortie (218).

2. Dispositif de pompe à membrane selon la revendication 1, **caractérisé en ce que** le siège de soupape de sortie (225) est un siège de soupape annulaire.

3. Dispositif de pompe à membrane selon la revendication 1 ou 2, **caractérisé en ce que** la soupape d'entrée (201) est une soupape de membrane qui présente un corps de soupape d'entrée dans lequel un évidement est réalisé, lequel est fermé pour la formation d'un espace de soupape d'entrée (217) par une membrane élastique (201), dans lequel un siège de soupape d'entrée (221) est agencé, dont le côté avant est tourné vers la membrane (201) et est agencé dans la position d'ouverture de la soupape d'entrée à distance de la membrane, dans lequel le siège de soupape d'entrée (221) est traversé par un canal de soupape d'entrée (222), et
le chemin d'afflux (219) comporte un premier canal d'afflux qui relie directement le raccord d'entrée (204) au canal de soupape d'entrée (222), et
un second canal d'afflux (213) qui relie l'espace de soupape d'entrée (217) refermable par la membrane élastique (201) à l'ouverture d'entrée (215) de la chambre de pompe (252).

4. Dispositif de pompe à membrane selon la revendication 3, **caractérisé en ce que** le siège de soupape d'entrée (221) est un siège de soupape annulaire.

5. Dispositif de pompe à membrane selon la revendication 3 ou 4, **caractérisé en ce que** le dispositif de pompe à membrane ou une partie du dispositif de pompe à membrane est réalisé(e) comme une cassette destinée à un usage unique qui est destinée à l'utilisation avec un dispositif d'actionnement (1) d'une pompe à membrane.

6. Dispositif de pompe à membrane selon la revendication 5, **caractérisé en ce que** le corps de chambre de pompe (252) et le corps de soupape d'entrée et de soupape de sortie (254A) font partie d'un corps de boîtier (254) à une partie ou plusieurs parties de la cassette, dans lequel la membrane (201) de la chambre de pompe et la membrane (201) de la soupape d'entrée et de sortie (202, 203) sont agencées au niveau d'une surface d'appui qui peut être couplée à une surface de montage (7) du dispositif d'actionnement (1).

7. Pompe à membrane avec un dispositif de pompe (2) selon l'une des revendications 3 à 6 et un dispositif d'actionnement (1) qui est réalisé de telle manière que la membrane (201) de la chambre de pompe (252) soit déformable entre une configuration d'aspiration et une configuration de décharge, dans laquelle la soupape d'entrée et de sortie (202, 203) est ouvrable et refermable en alternance de sorte que du fluide puisse être refoulé par la pompe à membrane.

8. Pompe à membrane selon la revendication 7, **caractérisé en ce que** le dispositif d'actionnement (1) présente un dispositif de réglage de sortie (14) qui présente un organe de réglage de sortie (38) pour la déformation de la membrane (201) de la soupape de sortie (203) qui est précontraint élastiquement dans une position de repos, dans laquelle la membrane de la soupape de sortie loge sur le siège de soupape de sortie (225) de sorte que la soupape de sortie soit fermée, dans laquelle l'organe de réglage de sortie est mobile de la position de repos dans une position fonctionnelle, dans laquelle la membrane de la soupape de sortie est agencée à distance du siège de soupape de sortie de sorte que la soupape de sortie soit ouverte,

9. Pompe à membrane selon la revendication 7 ou 8, **caractérisé en ce que** le dispositif d'actionnement (1) présente un dispositif de réglage d'entrée (9) qui présente un organe de réglage d'entrée (38) pour la déformation de la membrane (201) de la soupape d'entrée (202) qui est précontraint élastiquement dans une position de repos, dans laquelle la membrane de la soupape d'entrée loge sur le siège de soupape d'entrée (202) de sorte que la soupape d'entrée soit fermée, dans laquelle l'organe de réglage d'entrée est mobile de la position de repos dans une position fonctionnelle, dans laquelle la membrane de la soupape d'entrée est agencée à distance du siège de soupape d'entrée de sorte que la soupape d'entrée soit ouverte.

10. Pompe à membrane selon la revendication 9, **caractérisé en ce que** le dispositif d'actionnement (1) présente une première conduite de fluide de travail pour l'alimentation du dispositif de réglage d'entrée (9) avec un fluide de travail pour l'actionnement de l'organe de réglage d'entrée (38) et une seconde conduite de fluide de travail pour l'alimentation du dispositif de réglage de sortie (14) avec un fluide de travail pour l'actionnement de l'organe de réglage de sortie (38), et que le dispositif de réglage d'entrée (9) présente une première soupape de fluide de travail (81) pour l'influence d'une section transversale de la première conduite de fluide de travail et le dispositif de réglage de sortie (14) présente une seconde soupape de fluide de travail (82) pour l'influence d'une section transversale de la seconde conduite de fluide de travail, dans laquelle la première soupape de fluide de travail (81) est réalisée comme soupape de commutation et la seconde soupape de fluide de travail (82) est réalisée comme soupape proportionnelle.

11. Pompe à membrane selon la revendication 10, **caractérisé en ce que** le dispositif d'actionnement (1) présente un dispositif de commande (70) auquel les première et seconde soupapes de fluide de travail (81, 82) sont reliées électriquement, dans laquelle le dispositif de commande (70) est configuré de telle manière qu'en fonction du courant de masse de fluide du dispositif de pompe à membrane (2) une valeur de consigne de pression pour la seconde soupape de fluide de travail (82) soit déterminée de telle manière que le courant de masse de fluide soit constant pendant la phase de décharge du dispositif de pompe à membrane.

12. Pompe à membrane selon l'une des revendications 7 à 11, **caractérisé en ce que** le dispositif d'actionnement (1) présente une chambre de commande (8) et une conduite de fluide de commande pour la sollicitation de la chambre de commande avec un fluide de commande, dans laquelle une soupape de fluide de commande (74, 75) est agencée, laquelle est réalisée pour la modification d'une section transversale de la conduite de fluide de commande, dans laquelle la membrane (201) de la chambre de pompe (252) est déformable par sollicitation avec le fluide de commande dans une configuration d'aspiration ou une configuration de décharge.

13. Pompe à membrane selon la revendication 12, **caractérisé en ce qu'**un capteur de pression de fluide de commande (91) est prévu, lequel est relié électriquement au dispositif de commande (70), dans laquelle le dispositif de commande (70) est configuré de telle manière que le courant de masse de fluide soit généré en fonction du signal de pression du capteur de pression de fluide de commande (91),

14. Dispositif de traitement sanguin, en particulier dispositif de dialyse, avec un récipient de fourniture d'un liquide médical, en particulier une solution anticoagulation, et une pompe à membrane selon l'une des revendications 7 à 13 pour le refoulement du liquide médical.
